(19) Europäisches Patentamt
European Patent Office
Office européen des brevets

(11) **EP 1 551 359 B1**

(12) **EUROPEAN PATENT SPECIFICATION**

(45) Date of publication and mention
of the grant of the patent:
**16.09.2009 Bulletin 2009/38**

(21) Application number: **03753766.9**

(22) Date of filing: **10.10.2003**

(51) Int Cl.:
*A61K 8/365* (2006.01)    *A61Q 5/02* (2006.01)
*A61Q 17/00* (2006.01)    *A61Q 19/10* (2006.01)
*A01N 37/36* (2006.01)

(86) International application number:
**PCT/GB2003/004381**

(87) International publication number:
**WO 2004/032886 (22.04.2004 Gazette 2004/17)**

(54) **SURFACE TREATMENT**

OBERFLÄCHENBEHANDLUNG

TRAITEMENT DE SURFACE

(84) Designated Contracting States:
**AT BE BG CH CY CZ DE DK EE ES FI FR GB GR HU IE IT LI LU MC NL PT RO SE SI SK TR**

(30) Priority: **11.10.2002 GB 0223570**

(43) Date of publication of application:
**13.07.2005 Bulletin 2005/28**

(73) Proprietor: **Reckitt Benckiser (UK) Limited Slough,
Berkshire SL1 3UH (GB)**

(72) Inventors:
• **DUDDINGTON, A.,**
**Reckitt Benckiser Corp. Serv. Ltd.**
**Hull HU8 7DS (GB)**
• **FIELD, Paul F.,**
**Reckitt Benckiser Corp. Serv. Ltd.**
**Hull HU8 7DS (GB)**
• **PAYNE, D.N.,**
**Reckitt Benckiser Healthcare (UK) Ltd**
**Hull HU8 7DS (GB)**

(74) Representative: **Bowers, Craig Malcolm et al
Reckitt Benckiser
Corporate Services Limited
Legal Department - Patents Group
Dansom Lane
Hull
HU8 7DS (GB)**

(56) References cited:
EP-A- 0 727 204          WO-A-00/17303
WO-A-81/01516          WO-A-98/51264
US-B1- 6 262 038

• DATABASE WPI Section Ch, Week 199821
Derwent Publications Ltd., London, GB; Class
D16, AN 1998-233306 XP002267808 & HU 212 827
A (KELEMEN K), 28 July 1997 (1997-07-28)

**Description**

This invention relates to surface treatment compositions, and methods of treating surfaces. The invention relates particularly, but not exclusively, to compositions for treatment of the skin or hair to effect cleaning, and to related methods. For the purpose of this specification the term "cleaning" denotes removal of dirt (including grease) and/or combating of microorganisms.

Many compositions used for surface treatment include a surfactant or combination of surfactants which help to release dirt and/or microorganisms from the hard surface. Such compositions may also contain biocidal agents. Such compositions must be formulated carefully. For example surfactants may denature biocidal agents. Many biocidal agents have a detectable odour and the type and amount of a fragrance - typically an expensive ingredient - may need to be carefully selected. Foaming qualities are usually desired and these can be compromised by subtle aspects of the formulation selection. The viscosity of the compositions can be hard to control. The desired viscosity may be reduced as a function of time, temperature or pH. Optimal action of a biocidal agent in the composition may require a particular pH range, whereas a different pH range may be needed for optimal viscosity.

The nature and combination of surfactants chosen will also contribute significantly to the physical properties of such a formulation. Consideration of the solubilisation of biocidal agents in such systems is crucial, to obtain and maintain efficacy.

Frequently, a compromise is reached in known compositions in which either the viscosity or biocidal efficacy of the composition, or both, is sacrificed in part, in order to provide a composition which provides at least reasonable viscosity and biocidal activity. In many known compositions, the pH of the composition is raised or lowered in order to optimise the viscosity, or a combination of surfactants is utilised to promote thermodynamic instability, in order to increase efficacy. In some compositions where viscosity is not compromised, biocidal activity is less than optimal.

US-B1-6262038, EP-A-0727204, WO-A-9851264 and WO-A-0017303 each disclose compositions which contain surfactants and at least two organic acids which are usually citric and lactic acids. In particular, US-B1-6262038 discloses germicidal compositions for a variety of uses, such as on fruit and vegetables, skin and hair. The compositions contain a mixture of fruit acids including both citric and lactic acids, and biocidal surfactants such as sodium lauryl sulphate surfactant and ionic sophorose lipid surfactants.

It would therefore be advantageous to provide a surface treatment composition which provides a surfactant and biocidal action at optimal viscosity for good handling and high surfactant efficacy, but without reducing the efficacy of any biocidal agent(s) in the composition. It would also be advantageous to provide a composition which can be stored for relatively long periods of time without significant detrimental alteration of viscosity of the composition, or significant reduction in either surfactant effect or biocidal activity. Furthermore, it would be desirable to provide a surface treatment composition which includes a surfactant, the composition being at the optimum pH for good viscosity and viscosity maintenance, and good surfactant and biocidal action.

It is therefore an aim of preferred embodiments of the invention to overcome the problems of the prior art.

According to a first aspect of the present invention, there is provided a surface treatment composition comprising at least one surfactant and at least two different organic acids each present both as an organic acid and as an organic acid salt, and wherein the total concentration of the organic acids and salts of organic acids in the composition is at least 0.5% (w/v), wherein the composition further comprises a biocidal agent, which is a phenolic compound.

Preferred acids for use in the present invention are carboxylic acids. Preferred salts of organic acids for use with the present invention are carboxylates, preferably alkali metal carboxylates, more preferably potassium or, especially, sodium salts.

Preferred carboxylic acids/carboxylates are aliphatic; especially saturated aliphatic.

Other suitable organic acids and salts thereof may include benzene sulphonic acid and other aromatic sulphonic acids, uric acid and other purine-containing acids and ascorbic acid and other sugar-derived acids.

Suitably one of the organic acids or salt of an organic acid has two or more carboxylic acid or carboxylate groups.

In a preferred embodiment, one of said compounds is an organic acid and salt of the organic acid, having two or more carboxylic acid or carboxylate groups, and another of said compounds is an organic acid and salt of the organic acid, having one carboxylic acid or carboxylate group.

In another preferred embodiment, one of said compounds is an organic acid and a salt of an organic acid, having three carboxylic acid or carboxylate groups, and another of said compounds is an organic acid and a salt of an organic acid, having one or two carboxylic acid or carboxylate groups.

Suitable organic acids/salts with one carboxylic acid or carboxylate group include linear or branched optionally substituted hydroxyalkyl carboxylic acids/salts or alkylmonocarboxylic acids/salts, of 1 to 8 chain carbon atoms, preferably 1 to 6 chain carbon atoms.

A suitable monocarboxylic acid/salt is formic, acetic, chloroacetic, dichloroacetic, benzoic, 2,4,6-trihydroxybenzoic, 2-aminobenzoic, pyruvic, quinolinic, 2-chlorobenzoic, glyoxylic, thioacetic, glyceric, acetoacetic, hippuric, glycolic acid, and especially, lactic acid; or salts thereof.

Suitable organic acids/salts with two carboxylic acids or carboxylate groups include linear or branched optionally substituted hydroxyalkyldicarboxylic acids/salts or alkyldicarboxylic acids/salts, of 2 to 10 chain carbon atoms, preferably 2 to 8 chain carbon atoms. Preferred organic dicarboxylic acids/salts include tartaric, oxalic, maleic, aspartic, L-glutamic, oxaloacetic, 2-oxogluteric, malonic, phthalic, methylmalonic, mesaconic, methylsuccinic, glutaric, malic and adipic acids or salts thereof.

Suitable organic acids/salts with three carboxylic acids or carboxylate groups include linear or branched optionally substituted hydroxyalkyltricarboxylic acids/salts, alkyltricarboxylic acids/salts, of 3 to 10 chain carbon atoms, preferably 3 to 8 chain carbon atoms. Preferred organic tricarboxylic acids/salts include L-argininosuccinic, isocitric and, especially, citric acid or salts thereof.

In a particularly preferred embodiment of the invention the composition comprises two organic acids and salts of organic acids; preferably carboxylic acids/salts. Preferably one of the organic acids/salts comprises lactic acid and/or lactate and another of the organic acids/salts comprises citric acid and/or citrate.

The composition comprises both a first organic acid and a salt of that organic acid.

The composition comprises both a second organic acid and a salt of that organic acid.

Suitably the total concentration of all of the organic acids and salts thereof in the composition is at least 1% (w/v), preferably at least 2% (w/v).

Suitably the total concentration of both organic acids and salts thereof in the composition is no more than 10% (w/v), preferably no more than 7.5% (w/v).

Suitably the pH of the composition is no more than 6, preferably no more than 5.5, more preferably no more than 5 and most preferably no more than 4.8.

Suitably the pH of the composition is at least 2, preferably at least 3 and more preferably at least 4.

One or, preferably, all of the organic acids and/or salts preferably act to buffer the composition to a desired pH.

The surfactant may be anionic, cationic, non-ionic, zwitterionic or amphoteric.

There may be more than one surfactant, preferably being independently selected from an anionic, cationic, non-ionic, zwitterionic or amphoteric surfactant.

Suitable non-ionic surfactants include alkoxylated alcohols, particularly alkoxylated fatty alcohols. These include ethoxylated and propoxylated fatty alcohols, as well as ethoxylated and propoxylated alkyl phenols, both having alkyl groups of from 7 to 16, more preferably 8 to 13 carbon chains in length.

Examples of alkoxylated alcohols include certain ethoxylated alcohol compositions presently commercially available from the Shell Oil Company (Houston, TX) under the general trade name NEODOL (trade mark), which are described as linear alcohol ethoxylates, certain compositions presently commercially available from the Union Carbide Company, (Danbury, CT) under the general trade name TERGITOL (trade mark) which are described as secondary alcohol ethoxylates, and contain compositions present commercially available from Clariant (UK) under the general trade name GENAPOL (trade mark) and which are described to be linear and branched alcohol ethoxylates.

Examples of alkoxylated alkyl phenols include certain compositions presently commercially available from the Rhône-Poulenc Company (Cranbury, NJ) under the general trade name IGEPAL (trade mark), which are described as octyl and nonyl phenols.

Suitable anionic surfactants include linear $C_8$ to $C_{16}$ alkyl sulphates, $C_8$ to $C_{16}$ alkylsulphonates, $C_8$ to $C_{16}$ alkylbenzenesulphonates, $C_8$ to $C_{16}$ alkyldiphenyloxide disulphonates and C4 to C$_{16}$ alkylated naphthalene sulphonates. Suitable examples of anionic surfactants are sodium lauryl sulphonate and sodium dodecyl benezene sulphonate, or mixtures thereof. Preferably the anionic surfactant is selected from those comprising an alkali metal or ammonium cation.

A preferred composition of the present invention includes an anionic surfactant.

Suitable amphoteric surfactants include betaines.

A preferred composition of the present invention includes an amphoteric surfactant.

An especially preferred composition of the present invention includes an anionic surfactant in combination with an amphoteric surfactant. Preferably the ratio of the weight of the anionic surfactant to the weight of the amphoteric surfactant exceeds 1:1, and more preferably exceeds 2:1. Most preferably it exceeds 4:1. In highly preferred embodiments it exceeds 6:1.

Suitably the total concentration of the surfactant(s) in the composition is at least 2% (w/v), preferably at least 5% (w/v) and more preferably at least 8% (w/v).

Suitably the total concentration of the surfactant(s) in the composition is no more than 25% (w/v), preferably no more than 20% (w/v).

Suitably the composition is an aqueous composition. Preferably the composition comprises at least 50% (w/v) water, more preferably at least 60% (w/v), most preferably at least 70% (w/v).

The composition may comprise one or more further ingredients such as preservatives, thickeners, fragrance, chelating agents, and sodium chloride, for example.

The composition contains a biocidal agent which is a phenolic compound, such as PCMX.

There may be more than one biocidal agent present in the composition.

When a biocidal agent is present it may suitably be at a total concentration in the composition of at least 0.1% (w/v), preferably at least 0.2% (w/v) and more preferably at least 0.4% (w/v). Preferably it is present in an amount of up to 2% (w/v), more preferably up to 1% (w/v), most preferably up to 0.6% (w/v).

However it is believed that in preferred embodiments the acids and salts used in the invention may provide biocidal action, and it is possible that a traditional biocidal agent, such as an aromatic or heteroaromatic compound, notably a phenolic compound (for example PCMX), may not be needed in some embodiments. Accordingly compositions not containing such a biocidal agent are within the scope of the present invention.

Preferred compositions of the present invention have a foaming action with water on the surface to be treated.

According to a second aspect of the present invention there is provided a surface treatment composition comprising at least one surfactant and at least two different organic buffers.

Suitably the organic buffers comprise organic acids and salts thereof, as described above. Of course the buffers are selected to be compatible with each other in the composition, and compatible with other components of the composition.

Suitably the or each surfactant is as described for the first aspect of the invention.

Suitably the composition further comprises a biocidal agent as described for the first aspect of the invention.

Other definitions given above in relation the first aspect are applicable to the second aspect.

The composition of the first or second aspect is preferably a liquid skin cleaner (for example a hand wash), a shower gel, or the like.

In accordance with a third aspect of the present invention there is provided a package containing a composition of the first or second aspect, the package comprising a container for the composition and a restricted dispenser outlet therefrom under the control of a user. The restricted dispenser outlet could be, for example, a spray nozzle of a pressurised canister, or the outlet of a pump-action container, for example a press-action "tap" or the spray nozzle of a trigger spray container.

According to a fourth aspect of the invention there is provided a method of treating a surface, the method comprising the step of contacting the surface with the surface treatment composition of the first or second aspects of the invention.

Suitably the surface is a surface of a person, in particular the skin or hair of a person.

The method may comprise coating the surface with the composition, directly from a container or via the agency of a separate part, for example a sponge, cloth or the hand, or spraying the surface with the composition.

The method may comprise the final step of rinsing the surface with an aqueous media, suitably clean water.

Example

The various aspects of the invention will now be described with reference to the following non-limiting examples in which the following materials are used:

PCMX - parachloro meta-xylenol, supplied by Thomas Swan, Durham

EMPICOL ESB 70 (SLES) - sodium lauryl (C 12-16) ethoxy (2-3 EO) - sulphate surfactant, supplied by Huntsman

EMPIGEN BSFA - a betaine amphoteric surfactant, supplied by Huntsman

KATHON CG - a preservative; a mixture of thiazolinones, supplied by Rohn & Haas

JAGUAR EXCEL - a guar gum supplied by Rhodia

Pine fragrance

EMPICOL XPE/H - pearliser, supplied by Huntsman

EMPICOL, EMPIGEN, KATHON and JAGUAR EXCEL are trade marks.

A composition of the invention was made up according to Formulation A given in Table 1 below, in which lactic acid/sodium lactate and citric acid/sodium citrate were used as two different buffering agents. A second formulation, Formulation B was also prepared in which the citric acid/sodium citrate was omitted. A control formulation, Formulation C was prepared in which no such organic acid or salt was present.

Table 1

| Ingredient | Concentration (%w/v) | | |
|---|---|---|---|
| | Formulation A | Formulation B | Formulation C |
| PCMX | 0.5 | 0.5 | 0.5 |
| EMPICOL ESB 70 | 9.0 | 9.0 | 9.0 |
| EMPIGEN BSFA | 1.5 | 1.5 | 1.5 |
| Lactic acid | To pH 4.7 | To pH 4.7 | - |
| Sodium lactate | 1.0 | 1.0 | - |
| EMPICOL XPE/H | 1.5 | 1.5 | 1.5 |
| Fragrance | 0.2 | 0.2 | 0.2 |
| KATHON CG | 0.02 | 0.02 | 0.02 |
| Tetrasodium EDTA | 0.3 | 0.3 | 0.3 |
| Sodium citrate | 0.7 | - | - |
| Citric acid | To pH 4.7 | - | - |
| JAGUAR EXCEL | 0.3 | 0.3 | 0.3 |
| Sodium chloride | - | - | Q.S |
| Deionised water | to 100% | to 100% | to 100% |

The anti-microbial efficacy of Formulation A, Formulation B and Formulation C against *Staphylococcus aureus* (NCTC 10788) and *Escherichia coli* (NCTC 10418), was tested by performing a Handwash Efficacy Suspension Test.

The Handwash Efficacy Suspension Test is based on a standard.test for the assessment of the rapid germicidal activity for antibacterial liquid and bar soap products, test prEN12054 - chemical disinfection and antiseptics - Products for hygienic and surgical handrub and handwash, bactericidal activity, test method and requirements (phase 2, step 1); British Standard Institute draft for public comment 95/561926 July 1995; but with use of a different *E. coli* strain) .

The microbiocidal effect (ME) due to the action of the composition over the test contact time at the temperature at which the test was performed is expressed by the formula:

$$ME \quad = \quad Log\ N_C - Log\ N_D$$

where:

$N_C$ = Number of cfu/ml of the relevant control test (test mixture without composition).

$N_D$ = Number of cfu/ml of the test mixture after the action of the composition.

Results are graded as follows:

| ME values obtained | Activity |
|---|---|
| >4.0 | Excellent |
| 3.0 - 4.0 | Good |
| 1.5 - 3.0 | Moderate |
| 0.5 - 1.5 | Poor |
| <0.5 | No activity |

Formulations A, B and C were diluted in hard water to give a 50% v/v concentration, and were tested against S.aureus by contacting Formulations A, B and C with *S.aureus* cultures for one minute.

The tests were repeated a further two times to give a total of three repeats.

The results of the anti-microbial efficacy test against *S.aureus* are shown in Table 2.

Table 2

| Formulation | Replicate ME Values | | | Median ME Value |
|---|---|---|---|---|
| | Run 1 | Run 2 | Run 3 | |
| A | 2.68 | 2.48 | 3.08 | 2.68 |
| B | 0.95 | 0.78 | 2.23 | 0.95 |
| C | 1.38 | 1.25 | 1.61 | 1.38 |

The anti-microbial efficacy of Formulations A, B and C was tested against *Escherichia coli*, using the Handwash Efficacy Suspension Test as detailed above. The results of the test against *E. coli*) is shown in Table 3 below.

Table 3

| Formulation | Replicate ME Values | | | Median ME Value |
|---|---|---|---|---|
| | Run 1 | Run 2 | Run 3 | |
| A | 4.68 | 4.95 | 4.57 | 4.68 |
| B | 2.24 | 2.60 | 2.60 | 2.60 |
| C | 0.24 | 0.09 | 0.17 | 0.17 |

The results of the test as shown in Tables 2 and 3 show that Formulation A exhibited moderate activity against *S.aureus* with a median ME value of 2.68.

Formulation A also showed good activity against E. coli achieving a median ME value of 4.68. Formulation B showed moderate activity against E. coli compared to Formulation A, with median ME value of 2.6, whereas Formulation C without organic buffers showed no activity against this organism.

Viscosity stability issues were also studied. The polymeric thickener CROTHIX was found to be unstable at the pH of Formulation B (pH 4.7). The pH of the formulation was increased to pH 5.2 to avoid viscosity degradation over time. The microbial efficacy of the product decreased considerably as compared to Formulation B at pH 4.7. Also, after storage at 50°C for two weeks, the pH 5.2 formulation exhibited significant viscosity degradation. It was noticed that pH decreased over this time period. As such, an increase in buffering capacity of Formulation B was investigated by increasing the amount of sodium lactate/lactic acid pairing, which resulted in excess of sodium ions, and as a result, the formulation was not capable of thickening.

Conversely, Formulation A, employing namely sodium citrate/citric acid and sodium lactate/lactic acid, counteracted the loss of thickening capacity at pH 5.2, such that Formulation A at pH 5.2 showed significantly decreased viscosity degradation over time, with no significant loss of biocidal effect, compared to Formulation B in which the pH was increased by 5.2 by addition of further sodium lactate/lactic acid.

The biocidal efficacy of Formulation A at pH 5.2 was much higher (5 log reductions v. S.aureus and *E. coli*) compared to Formulation B at pH 5.2.

In further tests of a corresponding composition containing the citric and lactic buffer pairs bur not containing PCMX nor any other accepted biocidal agent, significant biocidal activity was still obtained.

## Claims

1. A surface treatment composition comprising at least one surfactant and in an amount of at least 0.5% (w/v) of at least two different organic acids each present both as an organic acid and as an organic acid salt, wherein the composition further comprises a biocidal agent, which is a phenolic compound.

2. A composition as claimed in Claim 1, wherein one of the organic acids or salt thereof has two or more carboxylic acid or carboxylate groups.

3. A composition as claimed in Claim 1 or 2, wherein one of the organic acids or salts thereof has two or more carboxylic acid or carboxylate groups, and the other organic acid or salt thereof has one carboxylic acid or carboxylate group.

4. A composition as claimed in any preceding claim, wherein one of the organic acids or salts thereof has one carboxylic acid or carboxylate group, being a linear or branched optionally substituted hydroxyalkyl carboxylic acid or salt or alkylmonocarboxylic acid or salt comprising 1 to 8 chain carbon atoms.

5. A composition as claimed in any preceding claim, wherein one of the organic acids or salts thereof has two carboxylic acid or carboxylate groups, and is a linear or branched optionally substituted hydroxyalkyldicarboxylic acid or salt thereof of 2 to 10 chain carbon atoms or is a linear or branched optionally substituted alkyldicarboxylic acid or salt thereof of 2 to 10 chain carbon atoms.

6. A composition as claimed in any preceding claim, wherein one of the organic acids or salts thereof has three carboxylic acids or carboxylate groups and is a linear or branched optionally substituted hydroxyalkyltricarboxylic acid or salt thereof of 3 to 10 chain carbon atoms or is a linear or branched optionally substituted alkyltricarboxylic acid or salt thereof of 3 to 10 chain carbon atoms.

7. A composition as claimed in any preceding claim, wherein at least one of the organic acids or salts thereof buffers the composition to a desired pH.

8. A composition according to claim 1, wherein the first acid is lactic acid and the salt of the first acid is a lactate, the second acid is citric acid and the salt of the second acid is a citrate.

9. A composition according to any preceding claim, wherein the total concentration of the organic acids and salts thereof present in the composition is at least 1% (w/v).

10. A composition as claimed in any preceding claim, wherein the pH of the composition is no more than 6.

11. A composition as claimed in any preceding claim, wherein the total concentration of surfactant(s) in the composition is at least 2% (w/v).

12. A surface treatment composition comprising a composition accoridng to any preceding claim.

13. A package comprising a container containing a composition according to any preceding claim, further comprising a restricted dispenser outlet under the control of a user.

14. A method of treating a surface, the method comprising the step of contacting the surface with a composition as claimed in any of claims 1 to 12.

15. A method as claimed in Claim 14, wherein the method is a method of removing dirt from a surface.

16. A method as claimed in Claim 14, wherein the method is a method of removing or inhibiting microbial growth from skin or hair.

**Patentansprüche**

1. Oberflächenbehandlungszusammensetzung, umfassend wenigstens einen oberflächenaktiven Stoff und in einer Menge von wenigstens 0,5% (w/v) wenigstens zwei verschiedene organische Säuren, die jeweils sowohl als organische Säure als auch als Salz der organischen Säure vorhanden sind, wobei die Zusammensetzung weiterhin ein biozides Mittel umfasst, das eine Phenolverbindung ist.

2. Zusammensetzung gemäß Anspruch 1, wobei eine der organischen Säuren oder das Salz derselben zwei oder mehr Carbonsäure- oder Carboxylatgruppen aufweist.

3. Zusammensetzung gemäß Anspruch 1 oder 2, wobei eine(s) der organischen Säuren oder Salze derselben zwei oder mehr Carbonsäure- oder Carboxylatgruppen, aufweist und die andere organische Säure oder das Salz derselben eine Carbonsäure- oder Carboxylatgruppe aufweist.

**4.** Zusammensetzung gemäß einem der vorhergehenden Ansprüche, wobei eine(s) der organischen Säuren oder Salze derselben eine Carbonsäure- oder Carboxylatgruppe aufweist und ein(e) lineare(s) oder verzweigte(s) optional substituierte(s) Hydroxyalkylcarbonsäure oder -salz oder Alkylmonocarbonsäure oder -salz ist, die bzw. das 1 bis 8 Ketten-Kohlenstoffatome umfasst.

**5.** Zusammensetzung gemäß einem der vorhergehenden Ansprüche, wobei eine(s) der organischen Säuren oder Salze derselben zwei Carbonsäure- oder Carboxylatgruppen aufweist und eine lineare oder verzweigte optional substituierte Hydroxyalkyldicarbonsäure oder ein Salz derselben mit 2 bis 10 Ketten-Kohlenstoffatomen ist oder eine lineare oder verzweigte optional substituierte Alkyldicarbonsäure oder ein Salz derselben mit 2 bis 10 Ketten-Kohlenstoffatomen ist.

**6.** Zusammensetzung gemäß einem der vorhergehenden Ansprüche, wobei eine(s) der organischen Säuren oder Salze derselben drei Carbonsäure- oder Carboxylatgruppen aufweist und eine lineare oder verzweigte optional substituierte Hydroxyalkyltricarbonsäure oder ein Salz derselben mit 3 bis 10 Ketten-Kohlenstoffatomen ist oder eine lineare oder verzweigte optional substituierte Alkyltricarbonsäure oder ein Salz derselben mit 3 bis 10 Ketten-Kohlenstoffatomen ist.

**7.** Zusammensetzung gemäß einem der vorhergehenden Ansprüche, wobei wenigstens eine(s) der organischen Säuren oder Salze derselben die Zusammensetzung auf einen gewünschten pH-Wert puffert.

**8.** Zusammensetzung gemäß Anspruch 1, wobei die erste Säure Milchsäure ist und das Salz der ersten Säure ein Lactat ist, die zweite Säure Zitronensäure ist und das Salz der zweiten Säure ein Citrat ist.

**9.** Zusammensetzung gemäß einem der vorhergehenden Ansprüche, wobei die Gesamtkonzentration der in der Zusammensetzung vorhandenen organischen Säuren und Salze derselben wenigstens 1% (w/v) beträgt.

**10.** Zusammensetzung gemäß einem der vorhergehenden Ansprüche, wobei der pH-Wert der Zusammensetzung nicht mehr als 6 beträgt.

**11.** Zusammensetzung gemäß einem der vorhergehenden Ansprüche, wobei die Gesamtkonzentration von oberflächenaktivem bzw. oberflächenaktiven Stoff(en) in der Zusammensetzung wenigstens 2% (w/v) beträgt.

**12.** Oberflächenbehandlungszusammensetzung, die eine Zusammensetzung gemäß einem der vorhergehenden Ansprüche umfasst.

**13.** Packung, umfassend einen Behälter, der eine Zusammensetzung gemäß einem der vorhergehenden Ansprüche enthält, weiterhin einen von einem Anwender gesteuerten eingeschränkten Abgabe-Auslass umfassend.

**14.** Verfahren zum Behandeln einer Oberfläche, wobei das Verfahren den Schritt umfasst, die Oberfläche mit einer Zusammensetzung gemäß einem der Ansprüche 1 bis 12 in Berührung zu bringen.

**15.** Verfahren gemäß Anspruch 14, wobei das Verfahren ein Verfahren zum Entfernen von Schmutz von einer Oberfläche ist.

**16.** Verfahren gemäß Anspruch 14, wobei das Verfahren ein Verfahren zum Entfernen oder Hemmen von mikrobiellem Wachstum an Haut oder Haar ist.

**Revendications**

**1.** Une composition de traitement de surface comprenant au moins un surfactant et dans un montant d'au moins 0,5 % (pds/vol) d'au moins deux acides organiques différents chacun présent, et comme un acide organique, et comme un sel d'acide organique, où la composition comprend de plus un agent biocide qui est un composant phénolique.

**2.** Une composition comme revendiqué en revendication 1 où un des acides organiques ou sel de celui-ci a deux ou plusieurs acides carboxyliques ou groupes carboxylates.

**3.** Une composition comme revendiqué en revendication 1 ou 2 où un des acides organiques ou sels de ceux-ci a deux ou plusieurs acides carboxyliques ou groupes carboxylates et l'autre acide organique ou sel de celui-ci a un acide carboxylique ou groupe carboxylate.

4.  Une composition comme revendiqué dans l'une des revendications précédentes où un des acides organiques ou sels de ceux-ci a un acide carboxylique ou groupe carboxylate, étant un acide carboxylique hydroxyalkyl ou sel éventuellement substitué linéaire ou ramifié ou acide alkylmonocarboxylique ou sel comprenant de 1 à 8 atomes de carbone en chaine.

5.  Une composition comme revendiqué dans l'une des revendications précédentes où un des acides organiques ou sels de ceux-ci a deux acides carboxyliques ou groupes carboxylates, et est un acide dicarboxylique hydroxyalkyl ou sel de celui-ci éventuellement substitué linéaire ou ramifié d'une chaîne de 2 à 10 atomes de carbone ou est un acide alkyldicarboxylique ou sel de celui-ci éventuellement substitué linéaire ou ramifié comprenant de 2 à 10 atomes de carbone en chaine.

6.  Une composition comme revendiqué dans l'une des revendications précédentes où un des acides organiques ou sels de ceux-ci a trois acides carboxyliques ou groupes carboxylates, et est un acide tricarboxylique, hydroxyalkyl ou sel de celui-ci éventuellement substitué linéaire ou ramifié d'une chaîne de 3 à 10 atomes de carbone ou est un acide alkyltricarboxylique ou sel de celui-ci éventuellement substitué linéaire ou ramifié comprenant de 3 à 10 atomes de carbone en chaine.

7.  Une composition comme revendiquée dans l'une des revendications précédentes où au moins un des acides organiques ou sels de ceux-ci tamponne la composition à un pH désiré.

8.  Une composition selon la revendication 1, où le premier acide lactique et le sel du premier acide est un lactate, le second acide est un acide citrique et le sel du second acide est un citrate.

9.  Une composition, selon l'une des revendications précédentes où la concentration totale des acides organiques et sels de ceux-ci présents dans la composition est d'au moins 1 % (pds/vol).

10. Une composition selon l'une des revendications précédentes où le pH de la composition n'est pas supérieur à 6.

11. Une composition selon l'une des revendications précédentes où la concentration totale de surfactant(s) dans la composition est d'au moins 2% (pds/vol).

12. Une composition de traitement de surface comprenant une composition selon l'une des revendications précédentes.

13. Un emballage comprenant un contenant contenant une composition selon l'une des revendications précédentes, comprenant de plus une sortie de distributeur restreinte sous le contrôle d'un utilisateur.

14. Un procédé de traitement d'une surface, le procédé comprenant l'étape d'entrée en contact de la surface avec une composition comme revendiqué dans l'une des revendications 1 à 12.

15. Un procédé comme revendiqué en revendication 14 où le procédé est un procédé d'enlèvement de saleté d'une surface.

16. Un procédé comme revendiqué en revendication 14 où le procédé est un procédé d'enlèvement ou d'inhibition de prolifération microbienne de la peau ou des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6262038 B1
- EP 0727204 A
- WO 9851264 A
- WO 0017303 A